(19) **Europäisches Patentamt**
**European Patent Office**
**Office européen des brevets**

(11) **EP 3 372 260 B1**

(12) **EUROPÄISCHE PATENTSCHRIFT**

(45) Veröffentlichungstag und Bekanntmachung des Hinweises auf die Patenterteilung:
**08.07.2020 Patentblatt 2020/28**

(51) Int Cl.:
***A61M 1/16*** *(2006.01)*

(21) Anmeldenummer: **18158427.7**

(22) Anmeldetag: **23.02.2018**

(54) **VORRICHTUNG UND VERFAHREN BEI EXTRAKORPORALER BLUTBEHANDLUNG**

DEVICE AND METHOD DURING EXTRACORPOREAL BLOOD TREATMENT

DISPOSITIF ET PROCÉDÉ PENDANT TRAITEMENT EXTRACORPOREL DU SANG

| | |
|---|---|
| (84) Benannte Vertragsstaaten:<br>**AL AT BE BG CH CY CZ DE DK EE ES FI FR GB GR HR HU IE IS IT LI LT LU LV MC MK MT NL NO PL PT RO RS SE SI SK SM TR** | (72) Erfinder: **Wagner, André**<br>**34117 Kassel (DE)** |
| (30) Priorität: **06.03.2017 DE 102017104634** | (74) Vertreter: **Winter, Brandl, Fürniss, Hübner, Röss, Kaiser, Polte - Partnerschaft mbB Patent- und Rechtsanwaltskanzlei Alois-Steinecker-Strasse 22 85354 Freising (DE)** |
| (43) Veröffentlichungstag der Anmeldung:<br>**12.09.2018 Patentblatt 2018/37** | |
| (73) Patentinhaber: **B. Braun Avitum AG**<br>**34212 Melsungen (DE)** | (56) Entgegenhaltungen:<br>**EP-A1- 3 156 086    DE-A1-102015 104 431**<br>**US-A1- 2005 000 868** |

EP 3 372 260 B1

**Beschreibung**

Die Erfindung betrifft eine Vorrichtung und Verfahren bei extrakorporaler Blutbehandlung und bezieht sich insbesondere auf eine Vorrichtung und ein Verfahren zur extrakorporalen bzw. bei extrakorporaler Blutbehandlung zum Erhalt eines Volumenentzugs unter Verwendung eines Bilanzkammerkreislaufs.

In der extrakorporalen Blutbehandlung und insbesondere der Dialyse hat eine Bilanzierungseinrichtung bzw. ein Bilanzierungssystem die Aufgabe, fertige Dialysierflüssigkeit kontinuierlich zu einem Dialysator und durch den Dialysator zu einem Auslauf zu transportieren. Die Bilanzierungseinrichtung muss dabei sicherstellen, dass das dem Dialysator zugeführte Flüssigkeitsvolumen identisch mit dem dem Dialysator entnommenen Flüssigkeitsvolumen ist. Zusätzlich ist eine Dosierungspumpe bzw. Ultrafiltrationspumpe dazu angeordnet, einem Patienten ein definiertes Ultrafiltrationsvolumen über die Dauer einer Therapie zu entnehmen (Volumenentzug). Dieses Ultrafiltrationsvolumen wird nicht von der Bilanzierungseinrichtung erfasst, so dass das entnommene Flüssigkeitsvolumen allein von der Ultrafiltrationspumpe abhängt.

Im Bereich bzw. bei der extrakorporalen Blutbehandlung sind membranlose Bilanzierungsverfahren sowie Bilanzierungsverfahren mit hochgenauen, wechselseitigen Pumpen bekannt.

Relevanter Stand der Technik ist zum Beispiel in den Dokumenten US2005/000868 A1, EP3156086 A1 und DE102015104431 A1 offenbart.

Eine beispielsweise bekannte membranlose Anordnung bzw. ein diese verwendendes Verfahren, die/das auf einer Coriolis-Messzelle basiert, nutzt mit elektromagnetischen Flussmessern oder Massendurchflussmessern den Coriolis-Effekt auf Grundlage der Trägheitskraft von Flüssigkeiten in schwingenden Rohrleitungen. Derartige Anordnungen können zwar genau sein, sind jedoch häufig störanfällig und insbesondere für die Anwendung in Dialysegeräten nicht in jedem Fall ausreichend stabil.

Eine andere, in Bezug auf wechselseitige Pumpen beispielsweise bekannte Anordnung bzw. ein entsprechendes Verfahren basiert auf einer Duplexpumpentechnik, bei welcher ein Bilanzierungssystem eine doppelt bereitgestellte Kolbenhubpumpe beinhaltet. Während eine der beiden Kolbenhubpumpen eine vorbestimmt bemessene Menge an Dialysierflüssigkeit zum Dialysator in ein geschlossenes System fördert, entzieht die zweite Kolbenhubpumpe dem geschlossenen System die exakt gleiche Menge verbrauchter Dialysierflüssigkeit. Nachteilig hierbei ist eine regelmäßig notwendige Wartung.

Ein bekanntes Bilanzierungssystem oder Bilanzkammersystem bzw. Bilanzierungskammersystem beinhaltet zwei Bilanzkammern, welche wechselseitig die Dialysierflüssigkeit dem Dialysator zuführen und abführen.

Im Wesentlichen sind in dem bekannten System eine erste Bilanzkammer und eine zweite Bilanzkammer angeordnet, von denen jede mit einer elastischen Membran in zwei Kammern unterteilt ist und zwei Zuläufe und zwei Abläufe aufweist, die jeweils mit einem Magnetventil geöffnet oder geschlossen werden können. Die Membranen der Bilanzkammern dehnen sich immer entgegengesetzt aus. Eine erste Flusspumpe transportiert verbrauchte Dialysierflüssigkeit in Richtung der ersten Bilanzkammer und drückt gleichzeitig frische Dialysierflüssigkeit zu einem Dialysator, und eine zweite Flusspumpe transportiert frische Dialysierflüssigkeit aus einer Dialysierflüssigkeitsaufbereitung des Geräts in Richtung der zweiten Bilanzkammer und drückt gleichzeitig verbrauchte Dialysierflüssigkeit in den Auslauf. Ein Dialysatdrucksensor ist dazu angeordnet, den Druck in der abfließenden Dialysierflüssigkeit nach dem Dialysator zu messen. Ferner ist ein Luftabscheider zur Abscheidung von Luft aus der Dialysierflüssigket angeordnet.

In einem geschlossenen Flüssigkeitskreislauf (geschlossenes System) für Dialysierflüssigkeit entspricht eine einlaufende Flüssigkeitsmenge genau einer auslaufenden Flüssigkeitsmenge. Nur die Ultrafiltrationspumpe (UF-Pumpe) kann diesem Kreislauf Flüssigkeit entziehen. In anderen Worten wird das eigentliche Ultrafiltrationsvolumen mittels einer (volumetrischen) Ultrafiltrationspumpe (beispielsweise einer Drehschieberkolbenpumpe), die dem geschlossenen Kreislauf Flüssigkeit entnimmt und damit die Ultrafiltration steuert, an der Bilanzkammer vorbeigeführt und sorgt somit für den eigentlichen Flüssigkeitsentzug des Patienten über die semipermeable Membran des Dialysators.

Da Bilanzkammersysteme und Ultrafiltrationspumpen gewisse Toleranzen aufweisen, kommt es nachteilig zu Abweichungen von der eingestellten Ultrafiltrationsmenge.

Der Erfindung liegt daher als eine Aufgabe zugrunde, eine Alternative zu dem bekannten System des Volumenentzugs mittels einer Ultrafiltrationspumpe bereitzustellen.

Erfindungsgemäß wird diese Aufgabe durch eine Vorrichtung bei extrakorporaler Blutbehandlung mit den Merkmalen des Anspruchs 1 und durch ein Verfahren bei extrakorporaler Blutbehandlung mit den Merkmalen des Anspruchs 7 gelöst.

Vorteilhafte Weiterbildungen der Erfindung sind Gegenstand der beigefügten Unteransprüche.

Gemäß einer zugrunde liegenden erfinderischen Idee kann durch Anordnen zweier Proportionaldruckventile im Zulauf und im Ablauf der Bilanzkammern durch eine gezielt einstellbare bzw. eingestellte Druckdifferenz zwischen den beiden Bilanzkammern durch die Ausdehnung der Materialien der Bilanzkammern ein Volumenentzug aus dem oder eine Volumenzufuhr in den Patienten erreicht werden.

Darüber hinaus kann durch einen Einsatz eines Heizelements oder eines Kühlelements, beispielsweise vermittels eines Wärmetauschers im Ablauf der Bilanzkammern, gezielt eine Temperaturdifferenz zwischen Bilanzkammerzulauf und Bilanzkammerablauf erzeugt werden, welcher wiederum aufgrund des Dichteunterschieds unterschiedlich warmer Flüssigkeiten in einem Volumenentzug aus dem oder einer Volumenzufuhr in den Patienten resultiert.

Auf die vorstehend beschriebene Art und Weise kann erfindungsgemäß die Ultrafiltrationspumpe im Bilanzkammerkreislauf eingespart oder zumindest umgangen werden, und kann zumindest der Flüssigkeitsentzug unter Entfall oder zumindest Umgehung der Ultrafiltrationspumpe realisiert werden. Die Erfindung stellt somit eine Alternative zu einem Volumenentzug auf der Grundlage des Betriebs einer Ultrafiltrationspumpe bereit, wobei die Ultrafiltrationspumpe weiterhin und beispielsweise umgehbar vorgesehen oder in Entfall gebracht sein kann.

Als vorteilhaft erweist sich erfindungsgemäß, dass die Ultrafiltrationspumpe konstruktiv entfallen kann, dass Fehlultrafiltrationen durch Dichteunterschiede und Druckunterschiede ausgeschlossen sind, dass ferner Konstantdruckventile konstruktiv entfallen können, dass ein Volumenentzug durch Kühlung durch einen Wärmetauscher gegen Wassereinlass möglich ist, und dass Anwendbarkeit bei sowohl Hämodialyse, Hämodiafiltration als auch Hämofiltration besteht.

Im Einzelnen wird die Aufgabe gelöst durch eine Vorrichtung bei extrakorporaler Blutbehandlung, die ein Bilanzierungssystem beinhaltet, wobei die Vorrichtung dazu angeordnet ist, ein Ultrafiltrationsvolumen als einen Volumenentzug auf der Grundlage einer Druckdifferenz und einer Temperaturdifferenz an einem Zulauf und einem Ablauf zumindest zweier Bilanzkammern des Bilanzierungssystems zu berechnen.

Bevorzugt beinhaltet die Vorrichtung bei extrakorporaler Blutbehandlung dazu ferner einen ersten Drucksensor und einen zweiten Drucksensor jeweils direkt hinter einem Zulauf bzw. Ablauf der zumindest zwei Bilanzkammern, wobei der erste Drucksensor und der zweite Drucksensor dazu angeordnet sind, einen Fluiddruck an ihrer Position zu bestimmen; einen ersten Temperatursensor und einen zweiten Temperatursensor an Eingängen der zumindest zwei Bilanzkammern, wobei der erste Temperatursensor und der zweite Temperatursensor dazu angeordnet sind, eine Temperatur im Zulauf und im Ablauf der Bilanzkammer zu bestimmen; und eine Berechnungseinrichtung zur Berechnung eines Ultrafiltrationsvolumens unter Verwendung der Druckdifferenz, die auf Grundlage von durch den ersten Drucksensor und den zweiten Drucksensor bestimmten Fluiddruckwerten ermittelbar ist, und der Temperaturdifferenz, die auf Grundlage von durch den ersten Temperatursensor und den zweiten Temperatursensor bestimmten Temperaturwerten ermittelbar ist.

Bevorzugt ist eine Ultrafiltrationspumpe in Entfall gebracht oder zumindest umgehbar angeordnet.

Bevorzugt sind ferner ein erstes Proportionalventil und ein zweites Proportionalventil dazu angeordnet, eine zum Erreichen eines Volumenentzugs erforderliche Druckdifferenz durch kontinuierliches Einstellen eines Drucks an jeweils den Positionen des ersten Drucksensors und des zweiten Drucksensors einzuregeln.

Bevorzugt sind das erste Proportionalventil und das zweite Proportionalventil dynamisch regelbar.

Bevorzugt ist ferner ein Heizelement und/oder Kühlelement, das zur Erzeugung und/oder Einregelung einer gezielten Temperaturdifferenz zwischen Bilanzkammerzulauf und Bilanzkammerablauf im Ablauf der Bilanzkammern angeordnet ist, wobei das Heizelement und/oder Kühlelement bevorzugt ein Wärmetauscher ist.

Die Berechnung des Volumenentzugs wird auf Grundlage der nachstehenden Formel durchgeführt:

$$UF_D = a * \Delta_P * DF_F + b * \Delta_T * DF_F$$

worin $UF_D$ das Ultrafiltrationsvolumen ist, $\Delta_P$ die Druckdifferenz ist, $\Delta_T$ die Temperaturdifferenz ist, a und b Koeffizienten sind, und $DF_F$ ein Dialysierflüssigkeitsvolumen ist.

Bevorzugt sind die Druckdifferenz und die Temperaturdifferenz proportional zu dem Ultrafiltrationsvolumen.

Erfindungsgemäß wird die Aufgabe auch gelöst durch ein Verfahren zur Berechnung eines Ultrafiltrationsvolumens als einen Volumenentzug in einer Vorrichtung bei extrakorporaler Blutbehandlung mit einem Bilanzierungssystem, beinhaltend den Schritt des Berechnens des Ultrafiltrationsvolumens auf der Grundlage einer Druckdifferenz und einer Temperaturdifferenz an einem Zulauf und einem Ablauf zumindest zweier Bilanzkammern des Bilanzierungssystems.

Die Erfindung wird nachstehend unter Bezugnahme auf die beigefügte Zeichnung näher beschrieben. Es zeigen:

Fig. 1 schematisch und ausschnittsweise einen Flussplan eines Bilanzierungssystems, das in einer Vorrichtung für extrakorporale Blutbehandlung bereitgestellt sein kann, gemäß einem Ausführungsbeispiel;

Fig. 2 schematisch ein Kennliniendiagramm einer Druckabhängigkeit eines Ultrafiltrationsvolumens gemäß dem Ausführungsbeispiel; und

Fig. 3 schematisch ein Kennliniendiagramm einer Temperaturabhängigkeit eines Ultrafiltrationsvolumens gemäß dem Ausführungsbeispiel.

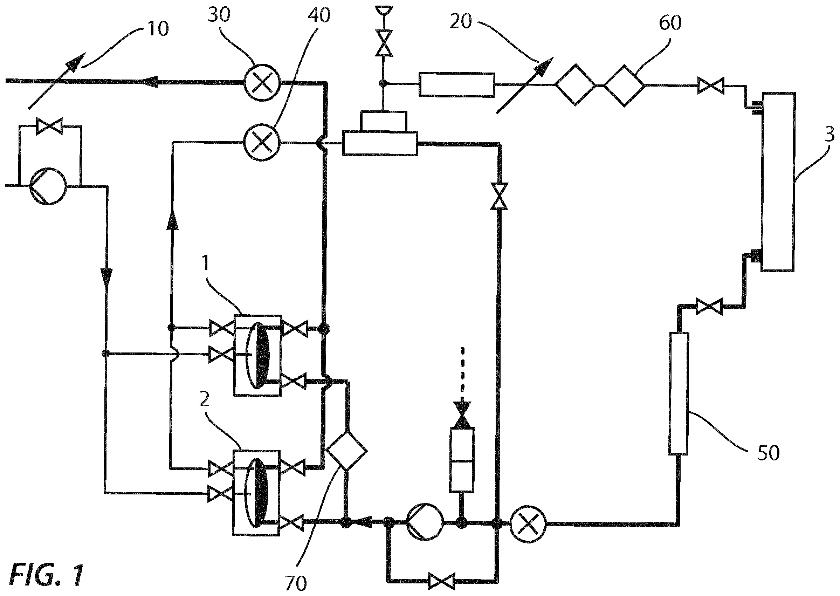

In Übereinstimmung mit Fig. 1 weist ein Bilanzierungssystem bzw. Bilanzkammersystem oder Bilanzierungskammersystem für extrakorporale bzw. bei extrakorporaler Blutbehandlung gemäß einem Ausführungsbeispiel, das Teil einer Vorrichtung zur extrakorporalen Blutbehandlung, wie beispielsweise einer Dialysemaschine, bilden kann, eine erste Bilanzkammer 1 und eine zweite Bilanzkammer 2 auf. Ein Dialysator ist mit dem Bezugszeichen 3 bezeichnet. An sich bekannte Komponenten des in Fig. 1 gezeigten Systems, die im Rahmen der Erfindung keine wesentliche Wirkung entfalten, sind nicht mit Bezugszeichen bezeichnet und zweckmäßigerweise nicht näher beschrieben.

Ein erster Drucksensor 30 (PDA2) und ein zweiter Drucksensor 40 (PDE) sind direkt hinter dem Zu- und Ablauf der Bilanzkammer 1, 2 dazu angeordnet, den Druck an ihrer Anordnungsposition bzw. dieser Stelle kontinuierlich zu bestimmen.

Ferner sind ein erstes Proportionalventil 10 (Prop1) und ein zweites Proportionalventil 20 (Prop2) dazu angeordnet, den Druck kontinuierlich auf Therapieerfordernisse (erforderliche Druckdifferenz zum Erreichen eines Volumenentzugs) einzustellen.

Das erste und das zweite Proportionalventil 10, 20 ersetzen dabei jeweils bekannte Konstantdruckventile und sind dynamisch regelbar.

Der Fluiddruck ist einerseits direkt von einer eingestellten Flussrate und andererseits von Alterungsbedingungen von Komponenten wie beispielsweise Dialysierflüssigkeitsfiltern abhängig. Zusätzlich wirken sich durch den venösen Rücklaufdruck (PV) oder den arteriellen Einlaufdruck (PBE, Druck Bluteintritt) bestimmte Druckänderungen auf der Blutseite ebenfalls direkt auf den Druck der Dialysierflüssigkeit bei Eintritt (PDE) aus.

Zusätzlich wird eine Temperaturdifferenz an den beiden Eingängen der Bilanzkammer(n) 1, 2 bestimmt. Die Temperaturdifferenz wird auf Grundlage einer Temperaturerfassung durch einen ersten Temperatursensor 70 (TSDA) und einen zweiten Temperatursensor 60 (TSD-S) ermittelt.

Wie in Fig. 1 dargestellt, sind der erste Drucksensor 30 und das erste Proportionalventil 10 in einem gemeinsamen Ablaufzweig stromab der Bilanzkammern 1, 2 (dickere Linienstärke) angeordnet, und sind der zweite Drucksensor 40 und das zweite Proportionalventil 20 in einem gemeinsamen Zulaufzweig (für den Zulauf frischer Dialysierflüssigkeit zum Dialysator 3, gleichbedeutend mit Ablaufzweig weg von den den Bilanzkammern 1, 2 und Zulaufzweig für die frische Dialysierflüssigkeit zum Dialysator 3 stromab der Bilanzkammern 1, 2, dünnere Linienstärke) angeordnet. Ferner ist der zweite Temperatursensor 70 in einem gemeinsamen Zulaufzweig stromauf der Bilanzkammern 1, 2 angeordnet (dickere Linienstärke), und ist der erste Temperatursensor 60 in demselben Fluidweg wie der zweite Drucksensor 40 und das zweite Proportionalventil 20 angeordnet (dünnere Linienstärke).

Die Anordnung der vorgenannten Komponenten in jeweils gemeinsamen Zweigen für beide Bilanzkammern 1, 2 resultiert beispielsweise daraus, dass die einzelnen Bilanzkammern zyklisch komplementär zueinander arbeiten (jeweils eine Bilanzkammer nimmt frische Dialysierflüssigkeit auf und drückt über die Membranverschiebung verbrauchte Dialysierflüssigkeit in den Ablauf, und die jeweils andere Bilanzkammer nimmt verbrauchte Dialysierflüssigkeit auf und drückt über die Membranverschiebung frische Dialysierflüssigkeit zum Dialysator 3), um einen kontinuierlichen Fluss im Dialysator 3 zu gewährleisten. Daher sind die Drucksensoren 30, 40, die Proportionalventile 10, 20 und die Temperatursensoren 60, 70 in Zweigabschnitten angeordnet, die von Fluid aus bzw. zu beiden Bilanzkammern 1, 2 durchflossen werden.

Im Abfluss (zu) der Bilanzkammer 1, 2, d.h. stromab des Dialysators 3, ist ferner ein Heizelement oder ein Kühlelement 50 angeordnet, über das auf eine zur Erzielung eines bestimmten Volumenentzugs benötigte, bestimmte Temperaturdifferenz regelbar bzw. steuerbar ist.

Eine Berechnung des Volumenentzugs durch die Druckdifferenz und die Temperaturdifferenz wird auf Grundlage der nachstehenden Formel (1) durchgeführt:

$$UF_D = a * \Delta_P * DF_F + b * \Delta_T * DF_F \qquad (1),$$

worin $UF_D$ das Ultrafiltrationsvolumen ist, $\Delta_P$ die Druckdifferenz ist, $\Delta_T$ die Temperaturdifferenz ist, a und b Koeffizienten sind, und $DF_F$ ein Dialysierflüssigkeitsvolumen ist.

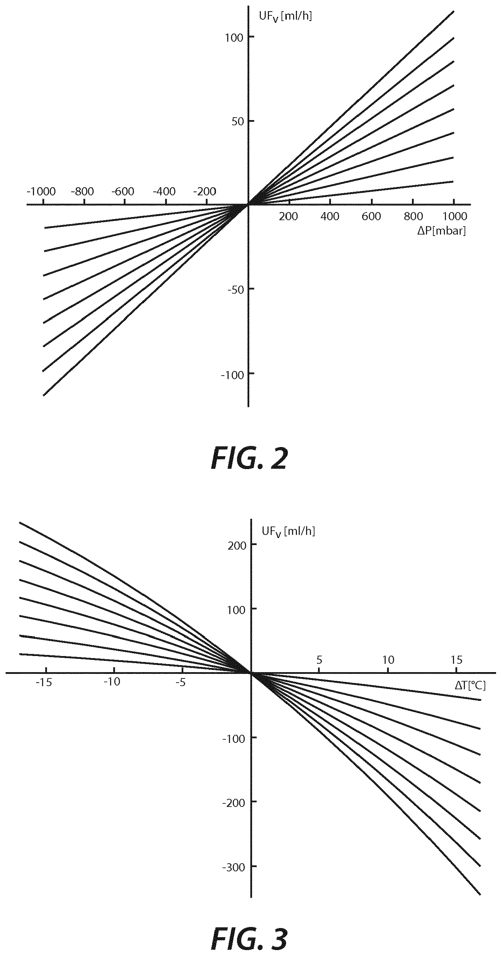

Die Druckdifferenz $\Delta_P$ und die Temperaturdifferenz $\Delta_T$ sind dabei proportional zu dem Ultrafiltrationsvolumen $UF_D$, wie beispielsweise in den Fig. 2 und 3 schematisch dargestellt.

Eine Berechnungseinrichtung zur Durchführung der vorgenannten Berechnung ist in verarbeitenden Komponenten der Vorrichtung, beispielsweise einer Steuereinrichtung, einer Prozessoreinrichtung und dergleichen, als Hardware und/oder Software integriert bzw. realisiert und als solche nicht weiter dargestellt.

In anderen Worten erfolgt gemäß dem Ausführungsbeispiel mittels einer vorbestimmten Regelung eine Einstellung einer erforderlichen Temperaturdifferenz und einer erforderlichen Druckdifferenz über jeweils die Proportionalventile 10, 20 sowie das Heizelement und/oder Kühlelement 50 im Abfluss, und wird ein entstehender Volumenentzug bei den resultierenden Temperatur- und Druckunterschieden berechnet.

Wie vorstehend beschrieben wurde, erfolgt eine Regelung des druckabhängigen und temperaturabhängigen Volumenentzugs auf der Grundlage einer Bestimmung der Druckverhältnisse vor und hinter der Bilanzkammer 1, 2 mittels eines Drucksensors für den Druck der Dialysierflüssigkeit bei Eintritt (PDE) und eines zweiten Drucksensors für den Druck der Dialysierflüssigkeit bei Austritt (PDA2), und auf der Grundlage einer Bestimmung der Temperaturverhältnisse mittels einer dazu in dem Gerät für extrakorporale Blutbehandlung, beispielsweise einer Dialysemaschine, verbauten und konfigurierten Temperatursensorik, bestehend aus einem Temperatursensor (TSD-S) und einem weiteren Temperatursensor (TSDA). Mit den vorgenannten Drucksensoren 30, 40 ist die Bestimmung des Druckunterschiedes vor und hinter der Bilanzkammer möglich.

Darüber hinaus ist sowohl im Zufluss als auch im Abfluss der Bilanzierungskammer ein Proportionalventil 10, 20 zur Druckregelung angeordnet, und ist zur Regelung der Temperaturdifferenz im Abfluss außerdem ein Heizelement oder ein Kühlelement 50, beispielsweise ein Wärmetauscher, bereitgestellt.

Bei dem vorstehend beschriebenen Bilanzierungssystem bzw. Bilanzkammersystem für extrakorporale Blutbehandlung kann somit eine Ultrafiltrationspumpe entfallen oder zumindest umgehbar installiert und konfiguriert sein.

Es versteht sich, dass die Erfindung nicht auf das vorstehend beschriebene Ausführungsbeispiel beschränkt ist, sondern durch die Ansprüche definiert ist. Beispielsweise ist denkbar, die vorstehend beschriebene Alternative zur Bestimmung des Volumenentzugs für bestehende Vorrichtungen mit lediglich einer Ultrafiltrationspumpe und gegebenenfalls Teilen der benötigten Sensorik und Regelung nachrüstbar zu gestalten. In diesem Fall kann es genügen, beispielsweise lediglich noch nicht vorhandene Teile der benötigten Sensorik und Regelung nachzuinstallieren und einen schaltbaren Bypass an der Ultrafiltrationspumpe anzuordnen.

Wie vorstehend beschrieben wurde, beinhaltet eine Vorrichtung bei extrakorporaler Blutbehandlung ein Bilanzierungssystem und berechnet ein Ultrafiltrationsvolumen ($UF_D$) als einen Volumenentzug auf Grundlage einer Druckdifferenz ($\Delta_P$) und einer Temperaturdifferenz ($\Delta_T$) an einem Zulauf und einem Ablauf zumindest zweier Bilanzkammern (1, 2). Dazu sind ein erster Drucksensor (30) und ein zweiter Drucksensor (40) jeweils direkt hinter einem Zulauf bzw. Ablauf von zwei Bilanzkammern (1, 2) angeordnet und bestimmen einen Fluiddruck an jeweils ihrer Position, und sind ein erster Temperatursensor (60) und ein zweiter Temperatursensor (70) an Eingängen der zumindest zwei Bilanzkammern (1, 2) angeordnet und bestimmen eine Temperatur im Zulauf und im Ablauf der Bilanzkammer. Das Ultrafiltrationsvolumens ($UD_F$) wird unter Verwendung der Druckdifferenz ($\Delta_P$), die auf Grundlage von durch den ersten Drucksensor (30) und den zweiten Drucksensor (40) bestimmten Fluiddruckwerten ermittelbar ist, und der Temperaturdifferenz ($\Delta_T$), die auf Grundlage von durch den ersten Temperatursensor (60) und den zweiten Temperatursensor (70) bestimmten Temperaturwerten ermittelbar ist, berechnet.

**Patentansprüche**

1. Vorrichtung zum Erhalt eines Volumenentzugs unter Verwendung eines Bilanzkammerkreislaufs bei extrakorporaler Blutbehandlung, beinhaltend:

   ein Bilanzierungssystem; wobei
   die Vorrichtung dazu angeordnet ist, ein Ultrafiltrationsvolumen ($UF_D$) als den Volumenentzug auf der Grundlage einer Druckdifferenz ($\Delta_P$) und einer Temperaturdifferenz ($\Delta_T$) an einem Zulauf und einem Ablauf zumindest zweier Bilanzkammern (1, 2) des Bilanzierungssystems zu berechnen, und
   ein erstes Proportionalventil (10) und ein zweites Proportionalventil (20) dazu angeordnet sind, die zum Erreichen des Volumenentzugs erforderliche Druckdifferenz durch kontinuierliches Einstellen eines Drucks an jeweils den Positionen eines ersten Drucksensors (30) und eines zweiten Drucksensors (40) einzuregeln.

2. Vorrichtung nach Anspruch 1, ferner beinhaltend:

   den ersten Drucksensor (30) und den zweiten Drucksensor (40) jeweils direkt hinter einem Zulauf bzw. Ablauf der zumindest zwei Bilanzkammern (1, 2), wobei der erste Drucksensor (30) und der zweite Drucksensor (40) dazu angeordnet sind, einen Fluiddruck an ihrer Position zu bestimmen;
   einen ersten Temperatursensor (60) und einen zweiten Temperatursensor (70) an Eingängen der zumindest zwei Bilanzkammern (1, 2), wobei der erste Temperatursensor (60) und der zweite Temperatursensor (70) dazu angeordnet sind, eine Temperatur im Zulauf und im Ablauf der Bilanzkammer zu bestimmen; und
   eine Berechnungseinrichtung zur Berechnung des Ultrafiltrationsvolumens ($UF_D$) unter Verwendung der Druckdifferenz ($\Delta_P$), die auf Grundlage von durch den ersten Drucksensor (30) und den zweiten Drucksensor (40) bestimmten Fluiddruckwerten ermittelbar ist, und der Temperaturdifferenz ($\Delta_T$), die auf Grundlage von durch den ersten Temperatursensor (60) und den zweiten Temperatursensor (70) bestimmten Temperaturwerten ermittelbar ist,
wobei
die Berechnung des Volumenentzugs auf Grundlage der nachstehenden Formel durchgeführt wird:

$$UF_D = a * \Delta_P * DF_F + b * \Delta_T * DF_F$$

worin $UF_D$ das Ultrafiltrationsvolumen ist, $\Delta_P$ die Druckdifferenz ist, $\Delta_T$ die Temperaturdifferenz ist, a und b Koeffizienten sind, und $DF_F$ ein Dialysierflüssigkeitsvolumen ist.

3. Vorrichtung nach Anspruch 1 oder 2, bei der eine Ultrafiltrationspumpe in Entfall gebracht oder zumindest umgehbar angeordnet ist.

4. Vorrichtung nach Anspruch 1, bei der das erste Proportionalventil (10) und das zweite Proportionalventil (20) dynamisch regelbar sind.

5. Vorrichtung nach einem der vorangehenden Ansprüche, ferner beinhaltend:
ein Heizelement und/oder Kühlelement (50), das zur Erzeugung und/oder Einregelung einer gezielten Temperaturdifferenz zwischen Bilanzkammerzulauf und Bilanzkammerablauf im Ablauf der Bilanzkammern (1, 2) angeordnet ist, wobei das Heizelement und/oder Kühlelement (50) bevorzugt ein Wärmetauscher ist.

6. Vorrichtung nach einem der vorangehenden Ansprüche, bei der die Druckdifferenz ($\Delta_P$) und die Temperaturdifferenz ($\Delta_T$) proportional zu dem Ultrafiltrationsvolumen ($UF_D$) sind.

7. Verfahren zur Berechnung eines Ultrafiltrationsvolumens ($UF_D$) als ein Volumenentzug in einer ein Bilanzierungssystem aufweisenden Vorrichtung zum Erhalt eines Volumenentzugs unter Verwendung eines Bilanzkammerkreislaufs bei extrakorporaler Blutbehandlung, beinhaltend die Schritte des
Berechnens des Ultrafiltrationsvolumens ($UF_D$) auf der Grundlage einer Druckdifferenz ($\Delta_P$), die mittels einer vorbestimmten Regelung und mittels eines ersten und eines zweiten Proportionalventils (10, 20) durch kontinuierliches Einstellen eines Drucks an jeweils den Positionen eines ersten und eines zweiten Drucksensors (30, 40) eingeregelt wurde, und einer Temperaturdifferenz ($\Delta_T$) an einem Zulauf und einem Ablauf zumindest zweier Bilanzkammern (1, 2) des Bilanzierungssystems, wobei
die Berechnung des Volumenentzugs auf Grundlage der nachstehenden Formel durchgeführt wird:

$$UF_D = a * \Delta_P * DF_F + b * \Delta_T * DF_F$$

worin $UF_D$ das Ultrafiltrationsvolumen ist, $\Delta_P$ die Druckdifferenz ist, $\Delta_T$ die Temperaturdifferenz ist, a und b Koeffizienten sind, und $DF_F$ ein Dialysierflüssigkeitsvolumen ist.

**Claims**

1. A device for extracorporeal blood treatment for obtaining a volume withdrawal using a balance chamber circuit, comprising:

    a balancing system; wherein
    the device is arranged to calculate an ultrafiltration volume ($UF_D$) as the volume withdrawal on the basis of a pressure difference ($\Delta_P$) and a temperature difference ($\Delta_T$) at an inlet and an outlet of at least two balance chambers (1, 2) of the balancing system, and
    a first proportional valve (10) and a second proportional valve (20) are arranged to control the pressure difference required for reaching the volume withdrawal by continuously adjusting a pressure at each of the positions of a first pressure sensor (30) and a second pressure sensor (40).

2. The device according to claim 1, further comprising:

the first pressure sensor (30) and the second pressure sensor (40) which are each arranged directly behind an inlet and an outlet of the at least two balance chambers (1, 2), respectively, the first pressure sensor (30) and the second pressure sensor (40) being arranged to determine a fluid pressure at their position;
a first temperature sensor (60) and a second temperature sensor (70) at inputs of the at least two balance chambers (1, 2), the first temperature sensor (60) and the second temperature sensor (70) being arranged to determine a temperature in the inlet and the outlet of the balance chamber; and
a calculation device for calculating the ultrafiltration volume ($UF_D$) using the pressure difference ($\Delta_P$) which can be established on the basis of fluid pressure values determined by the first pressure sensor (30) and the second pressure sensor (40), and the temperature difference ($\Delta_T$) which can be established on the basis of temperature values determined by the first temperature sensor (60) and the second temperature sensor (70),
wherein
the calculation of the volume withdrawal is carried out on the basis of the following formula:

$$UF_D = a * \Delta_P * DF_F + b * \Delta_T * DF_F$$

wherein $UF_D$ is the ultrafiltration volume, $\Delta_P$ is the pressure difference, $\Delta_T$ is the temperature difference ($\Delta_T$), a and b are coefficients, and $DF_F$ is a dialysis liquid volume.

3. The device according to claim 1 or 2, in which an ultrafiltration pump (10) is omitted or at least arranged such that it can be bypassed.

4. The device according to claim 1, in which the first proportional valve (10) and the second proportional valve (20) are dynamically controllable.

5. The device according to one of the preceding claims, further comprising:
a heating element and/or cooling element (50) which is/are arranged in the outlet of the balance chambers (1, 2) for creating and/or controlling a targeted temperature difference between the balance chamber inlet and balance chamber outlet, wherein the heating element and/or cooling element (50) preferably is a heat exchanger.

6. The device according to one of the preceding claims, in which the pressure difference ($\Delta_P$) and the temperature difference ($\Delta_T$) are proportional to the ultrafiltration volume ($UF_D$).

7. A method for calculating an ultrafiltration volume ($UF_D$) as a volume withdrawal in a device for obtaining a volume withdrawal using a balance chamber circuit for extracorporeal blood treatment comprising a balancing system, comprising the steps of
calculating the ultrafiltration volume ($UF_D$) on the basis of a pressure difference ($\Delta_P$) which was regulated by means of a predetermined control process and by means of a first and a second proportional valve (10, 20) by continuously adjusting a pressure at the positions of a first and a second pressure sensor (30, 40) and a temperature difference ($\Delta_T$) at an inlet and an outlet of at least two balance chambers (1, 2) of the balancing system, wherein
the calculation of the volume withdrawal is carried out on the basis of the following formula:

$$UF_D = a * \Delta_P * DF_F + b * \Delta_T * DF_F$$

wherein $UF_D$ is the ultrafiltration volume, $\Delta_P$ is the pressure difference, $\Delta_T$ is the temperature difference, a and b are coefficients, and $DF_F$ is a dialysis liquid volume.

**Revendications**

1. Dispositif pour obtenir une réduction de volume au moyen d'un circuit de chambre d'équilibrage lors d'un traitement extracorporel du sang, incluant :

un système d'équilibrage ; dans lequel
le dispositif est disposé en outre pour calculer un volume d'ultrafiltration ($UF_D$) en tant que réduction de volume sur la base d'une différence de pression ($\Delta_P$) et d'une différence de température ($\Delta_T$) au niveau d'une arrivée et d'une évacuation d'au moins deux chambres d'équilibrage (1, 2) du système d'équilibrage, et une première soupape proportionnelle (10) et une seconde soupape proportionnelle (20) sont disposées pour réguler la différence de pression nécessaire pour obtenir la réduction de volume par réglage continu d'une pression au niveau respectivement des positions d'un premier capteur de pression (30) et d'un second capteur de pression (40).

2. Dispositif selon la revendication 1, incluant en outre :

le premier capteur de pression (30) et le second capteur de pression (40) respectivement directement derrière une arrivée ou évacuation des aux moins deux chambres d'équilibrage (1, 2), dans lequel le premier capteur de pression (30) et le second capteur de pression (40) sont disposés pour définir une pression de fluide au niveau de leur position ;
un premier capteur de température (60) et un second capteur de température (70) aux entrées des au moins deux chambres d'équilibrage (1, 2), dans lequel le premier capteur de température (60) et le second capteur de température (70) sont disposés pour définir une température dans l'arrivée et dans l'évacuation de la chambre d'équilibrage ; et
un système de calcul pour calculer le volume d'ultrafiltration ($UF_D$) au moyen de la différence de pression ($\Delta_P$), qui peut être déterminée sur la base de valeurs de pression de fluide définies par le premier capteur de pression (30) et le second capteur de pression (40), et la différence de température ($A_T$), qui peut être déterminée sur la base de valeurs de température définies par le premier capteur de température (60) et le second capteur de température (70),
dans lequel
le calcul de la réduction de volume est réalisé sur la base de la formule suivante :

$$UF_D = a * \Delta_P * DF_F + b * \Delta_T * DF_F$$

dans laquelle $UF_D$ est le volume d'ultrafiltration, $\Delta_P$ est la différence de pression, $\Delta_T$ est la différence de température, a et b sont des coefficients, et $DF_F$ est un volume de liquide de dialyse.

3. Dispositif selon la revendication 1 ou 2, où une pompe d'ultrafiltration n'est plus nécessaire ou est disposée de manière à pouvoir au moins être contournée.

4. Dispositif selon la revendication 1, où la première soupape proportionnelle (10) et la seconde soupape proportionnelle (20) peuvent être régulées de manière dynamique.

5. Dispositif selon l'une quelconque des revendications précédentes, incluant en outre :
un élément de chauffage et/ou élément de refroidissement (50), qui, pour générer et/ou réguler une différence de température ciblée entre l'arrivée de chambre d'équilibrage et l'évacuation de chambre d'équilibrage, est disposé dans l'évacuation des chambres d'équilibrage (1, 2), dans lequel l'élément de chauffage et/ou l'élément de refroidissement (50) est de préférence un échangeur de chaleur.

6. Dispositif selon l'une quelconque des revendications précédentes, où la différence de pression ($\Delta_P$) et la différence de température ($\Delta_T$) sont proportionnelles au volume d'ultrafiltration ($UF_D$).

7. Procédé de calcul d'un volume d'ultrafiltration ($UF_D$) en tant que réduction de volume dans un dispositif présentant un système d'équilibrage, destiné à obtenir une réduction de volume, au moyen d'un circuit de chambre d'équilibrage lors d'un traitement extracorporel du sang, incluant les étapes de
calcul du volume d'ultrafiltration ($UF_D$) sur la base d'une différence de pression ($\Delta_P$), qui a été régulée au moyen d'une régulation prédéfinie et au moyen d'une première et d'une seconde soupape proportionnelle (10, 20) par réglage continu d'une pression au niveau respectivement des positions d'un premier et d'un second capteur de pression (30, 40), et d'une différence de température ($\Delta_T$) au niveau d'une arrivée et d'une évacuation d'au moins deux chambres d'équilibrage (1, 2) du système d'équilibrage, dans lequel
le calcul de la réduction de volume est réalisé sur la base de la formule suivante :

$$UF_D = a * \Delta_P * DF_F + b * \Delta_T * DF_F$$

dans laquelle UF$_D$ est le volume d'ultrafiltration, $\Delta_P$ est la différence de pression, $\Delta_T$ est la différence de température, a et b sont des coefficients, et DF$_F$ est un volume de liquide de dialyse.

FIG. 1

FIG. 2

FIG. 3

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 2005000868 A1
- EP 3156086 A1
- DE 102015104431 A1